# FASCICULE DE BREVET EUROPEEN

(11) **EP 2 198 923 B1**
(45) Date de publication et mention de la délivrance du brevet: **29.07.2015**
(21) Numéro de dépôt: 09179779.5
(22) Date de dépôt: 18.12.2009
(51) Int. Cl.: A61Q 5/08, A61Q 5/10, A61K 8/22, A61K 8/31, A61K 8/34, A61K 8/37, A61K 8/39, A61K 8/42, A61K 8/45, A61K 8/86, A61K 8/92

(54) **Composition oxydante pour le traitement des fibres kératiniques comprenant une huile, un alcool gras et un alcool gras oxyalkylène**
Oxidative Zusammensetzung zur Behandlung von Keratinfasern enthaltend ein Öl, einen Fettalkohol und einen alkoxylierten Fettalkohol
Oxidizing composition for the treatment of keratin fibres comprising an oil, a fatty alcohol and an oxyalkylenated fatty alcohol

(30) Priorité: 19.12.2008 FR 0858840
(43) Date de publication de la demande: 23.06.2010
(73) Titulaire: L'Oréal, 75008 Paris (FR)
(72) Inventeur: Braida-Valerio, Damarys, 75012, Paris (FR); Simonet, Frédéric, 92110, Clichy (FR); Nicolas-Morgantini, Luc, 60810, Rully (FR)
(74) Mandataire: Prevel, Estelle Nicole

(56) Documents cités:
- EP-A- 1 707 184
- EP-A- 2 005 939
- EP-A2- 1 321 132
- WO-A-01/41723
- WO-A2-03/053329
- DE-A1-102005 011 459
- DE-C1- 19 815 338
- JP-A- 2003 081 792
- US-A1- 2004 226 110
- US-A1- 2006 242 773
- US-B1- 6 238 653

## Description

La présente invention a pour objet une composition oxydante pour la coloration ou la décoloration des fibres kératiniques, et en particulier des fibres kératiniques humaines telles que les cheveux, comprenant, dans un milieu cosmétiquement acceptable, un ou plusieurs alcools gras ; un ou plusieurs alcools gras oxyalkylénés ; un ou plusieurs agents oxydants; au moins 10 % en poids du poids total de la composition d'une ou plusieurs huiles différentes des alcools gras ; et un ou plusieurs amides d'acides gras en C₈-C₃₀ éventuellement oxyalkylénées ; le rapport pondéral alcools gras / alcools gras oxyalkylénés étant inférieur ou égal à 5, le pH de la composition étant compris entre 1,5 et 4,5.

En cosmétique, dans les domaines de la teinture et de la décoloration des fibres kératiniques, et en particulier des fibres kératiniques humaines telles que les cheveux, on utilise des compositions oxydantes.

Ainsi, en teinture d'oxydation des cheveux, des compositions oxydantes sont mélangées aux colorants d'oxydation (bases et coupleurs), qui sont incolores par eux-mêmes, pour engendrer des composés colorés et colorants par un processus de condensation oxydative. Des compositions oxydantes sont également utilisées en teinture directe des cheveux en mélange avec certains colorants directs qui sont colorés et colorants pour obtenir une coloration avec un effet éclaircissant des cheveux. Parmi les agents oxydants classiquement utilisés pour la teinture des fibres kératiniques, on peut citer le peroxyde d'hydrogène ou des composés susceptibles de produire le peroxyde d'hydrogène par hydrolyse tels que le peroxyde d'urée. On peut aussi utiliser les persels comme les perborates et persulfates. Le peroxyde d'hydrogène est plus particulièrement préféré.

En décoloration des cheveux, les compositions de décoloration contiennent un ou plusieurs agents oxydants. Parmi ces agents oxydants, les plus classiquement utilisés sont le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés.

Ces compositions peuvent être des compositions aqueuses contenant des agents alcalins (amines ou ammoniaque) que l'on dilue au moment de l'emploi avec une composition aqueuse de peroxyde d'hydrogène.

Ces compositions peuvent aussi être formées de produits anhydres qui contiennent des composés alcalins (amines et silicates alcalins), et un réactif peroxygéné tels que les persulfates, perborates ou percarbonates, d'ammonium ou de métaux alcalins, que l'on dilue au moment de l'emploi avec une composition aqueuse de peroxyde d'hydrogène. En déformation permanente des cheveux, dans un premier temps, on réalise l'ouverture des liaisons disulfures -S-S- de la kératine (cystine) à l'aide d'une composition contenant un agent réducteur adapté (étape de réduction) puis, après avoir rincé la chevelure ainsi traitée, on reconstitue dans un second temps les liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou des shampooings, et ceci par opposition aux simples techniques classiques de déformation temporaire, telles que de mise en pli.

Les compositions oxydantes nécessaires à la mise en oeuvre de l'étape de fixation, sont le plus souvent des compositions à base d'eau oxygénée. Le document EP1707184 décrit des compositions colorantes comprenant un agent oxydant, un alcool gras, des tensioactifs et un faible taux d'huile.

L'introduction d'une teneur importante en huile, en remplacement de l'eau, dans les compositions oxydantes de coloration et / ou de décoloration ou de déformation permanente des fibres kératiniques permet d'améliorer les performances des actifs. Cependant, l'introduction d'une teneur importante en huile dans la composition oxydante conduit à une déstabilisation de la composition qui déphase au bout de quelques jours.

Le but de la présente invention est de fournir de nouvelles compositions oxydantes qui permettent d'améliorer les propriétés des compositions de coloration et / ou de décoloration et qui sont stables dans le temps. Ce but est atteint avec la présente invention qui a pour objet une composition pour la coloration ou la décoloration des fibres kératiniques, et en particulier des fibres kératiniques humaines telles que les cheveux, comprenant, dans un milieu cosmétiquement acceptable :
- un ou plusieurs alcools gras ;
- un ou plusieurs alcools gras oxyalkylénés ;
- un ou plusieurs agents oxydants ;
- au moins 10 % en poids du poids total de la composition d'une ou plusieurs huiles différentes des alcools gras ;
et un ou plusieurs amides d'acides gras en C₈-C₃₀ éventuellement oxyalkylénées ;
le rapport pondéral alcools gras / alcools gras oxyalkylénés étant inférieur ou égal à 5 ; le pH de la composition étant compris entre 1,5 et 4,5.

Lorsque la composition conforme à l'invention est utilisée pour la coloration des fibres kératiniques, on obtient de bonnes propriétés tinctoriales, notamment des colorations puissantes, chromatiques, peu sélectives et résistant bien aux diverses agressions que peuvent subir les cheveux telles que les shampooings, la lumière, la sueur et les déformations permanentes, sans altérer les propriétés cosmétiques des fibres kératiniques.

Lorsque la composition conforme à la présente invention est utilisée pour la décoloration des fibres kératiniques, elle permet d'obtenir un bon effet d'éclaircissement de ces fibres sans les dégrader et sans altérer leurs propriétés cosmétiques.

De plus, la composition conforme à l'invention présente une bonne stabilité dans le temps, notamment au stockage à des températures élevées, par exemple de l'ordre de 45 °C.

L'invention a aussi pour objet un procédé de coloration ou de décoloration des fibres kératiniques, mettant en oeuvre cette composition oxydante.

Un autre objet de l'invention est l'utilisation de cette composition oxydante pour la coloration ou la décoloration des fibres kératiniques.

Dans ce qui suit, sauf indication contraire, les bornes des intervalles indiqués sont comprises dans l'invention.

Le ou les alcools gras présents dans la composition conforme à l'invention peuvent être choisis parmi les alcools non (poly)oxyalkylénés (l'alkyle ayant 1 à 3 atomes de carbone) et non (poly)glycérolés, comprenant une ou plusieurs chaînes grasses présentant de 10 à 30 atomes de carbone, plus particulièrement de 14 à 22 atomes de carbone et de façon encore plus avantageuse, de 16 à 18 atomes de carbone, saturés ou insaturés, les chaînes grasses étant éventuellement substituées par un ou deux groupements hydroxyle supplémentaires. Lorsque l'alcool est insaturé, il comprend de 1 à 3 doubles liaisons carbone-carbone (-C=C-), conjuguées ou non. De préférence, l'alcool gras est un monoalcool.

A titre d'exemples d'alcools gras, on peut citer l'alcool laurique, cétylique, stéarylique, béhénique, myristique, linoléïque, undécylénique, palmitoléïque, linolénique, arachidonique, érucique, isocétylique, isostéarylique, isobéhénylique, l'alcool oléique et leurs mélanges.

De préférence, la composition comprend un ou plusieurs monoalcools gras non (poly)oxyalkylénés et non (poly)glycérolés, comprenant de 14 à 22 atomes de carbone et plus précisément, de 16 à 18 atomes de carbone, saturés.

La composition conforme à l'invention présente généralement une teneur en alcool(s) gras comprise entre 3 et 25 % en poids par rapport au poids de la composition, de préférence entre 5 et 20 % en poids et, conformément à une variante encore plus préférée de l'invention, de 6 à 18 % en poids.

Le ou les alcools gras oxyalkylénés peuvent être choisis parmi les composés de formule (I) suivante : 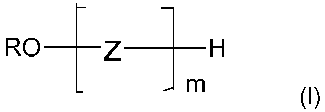 dans laquelle :
R désigne un radical saturé ou insaturé, linéaire ou ramifié, comportant de 8 à 40 atomes de carbone, et de préférence de 8 à 30 ;
Z représente un radical oxyéthyléné (i) et / ou oxypropyléné (ii)1 et (ii)2 de formules respectives suivantes :

   -CH₂-CH₂-O (i)

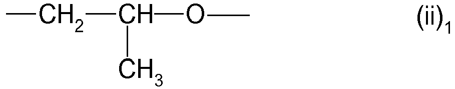

   -CH₂-CH₂-CH₂-O- (ii)₂ ;
m représente le nombre de groupements oxyde d'éthylène (i) et / ou oxyde de propylène (ii)1 ou (ii)2, allant de 1 et 250, et de préférence de 2 à 100.

Des alcools gras oxyalkylénés particulièrement préférés selon l'invention sont des alcools gras saturés ou non, linéaires ou ramifiés, comportant de 10 à 20 atomes de carbone et 2 à 40 groupements oxyde d'éthylène.

Comme composés de type alcool gras oxyalkyléné, on peut notamment citer les produits commercialisés suivants :
Mergital LM2 (COGNIS) [alcool laurique 2 OE] ;
Ifralan L12 (IFRACHEM) et Rewopal 12 (GOLDSCHMITDT) [alcool laurique 12 OE] ;
Empilan KA 2.5/90FL (ALBRIGHT & WILSON) et Mergital BL309 (COGNIS) [alcool décylique 3 OE] ;
Empilan KA 5/90FL (ALBRIGHT & WILSON) et Mergital BL589 (COGNIS) [alcool décylique 5 OE] ;
Brij 58 (UNIQUEMA) et Simulsol 58 (SEPPIC) [alcool cétylique 20 OE] ;
Eumulgin 05 (COGNIS) [alcool oleocétylique 5 OE] ;
Mergital OC30 (COGNIS) [alcool oleocétylique 30 OE] ;
Brij 72 (UNIQUEMA) [alcool stéarylique 2 OE] ;
Brij 76 (UNIQUEMA) [alcool stéarylique 10 OE] ;
Brij 78P (UNIQUEMA) [alcool stéarylique 20 OE] ;
Brij 700 (UNIQUEMA) [alcool stéarylique 100 OE];
Eumulgin B1 (COGNIS) [alcool cétylstéarylique 12 OE] ;
Eumulgin L (COGNIS) [alcool cétylique 9 OE et 2 OP] ;
Witconol APM (GOLDSCHMIDT) [alcool myristique 3 OP].

Le ou les alcools gras oxyalkylénés sont généralement présents en quantité comprise entre 0,05 et 50 %, et de préférence entre 0,5 et 40 %, et encore plus préférentiellement entre 1 et 20 % en poids du poids total de la composition.

Le ou les agents oxydants présents dans la composition conforme à l'invention peuvent être choisis parmi le peroxyde d'hydrogène, le peroxyde d'urée, les bromates alcalins, les polythionates, les persels tels que les perborates, les percarbonates et les persulfates.

De préférence, l'agent oxydant est le peroxyde d'hydrogène.

Le ou les agents oxydants représentent généralement de 0,1 à 50 %, de préférence de 1 à 20 % en poids par rapport au poids total de la composition oxydante.

Selon un mode de réalisation particulier de l'invention, lorsque l'agent oxydant est le peroxyde d'hydrogène, la composition oxydante comprend un ou plusieurs agents stabilisants de l'eau oxygénée.

A titre d'exemples d'agents stabilisants de l'eau oxygénée, on peut citer en particulier les pyrophosphates des métaux alcalins ou alcalino-terreux tels que le pyrophosphate de tétrasodium, les stannates des métaux alcalins ou alcalino-terreux, la phénacétine ou les sels d'acides et d'oxyquinoléine comme le sulfate d'oxyquinoléine. De préférence, on utilise un ou plusieurs stannates en association ou non avec un ou plusieurs pyrophosphates.

Le ou les agents stabilisants de l'eau oxygénée représentent généralement de 0,0001 à 5 % en poids, et de préférence de 0,01 à 2 % en poids par rapport au poids total de la composition oxydante.

Dans la composition conforme à l'invention, le rapport pondéral alcools gras / alcools gras oxyéthylénés est inférieur ou égal à 5, et de préférence de 0,1 à 5, encore plus préférentiellement de 0,5 à 4, mieux de 1 à 3.

La composition contient une ou plusieurs huiles différentes des alcools gras de l'invention. Ces huiles sont présentes dans la composition à une teneur supérieure ou égale à 10 %, encore plus préférentiellement supérieure ou égale à 15 %, mieux encore supérieure ou égale à 20 % en poids du poids total de la composition.

La concentration en huiles peut ainsi aller de 10 à 60 %, plus préférentiellement de 15 à 55 %, mieux de 20 à 50 % en poids du poids total de la composition.

Au sens de la présente invention, on entend par huile tout composé liquide à 25 °C et à la pression atmosphérique (760 mm de mercure) insoluble dans ces conditions à 5 % en poids, et de préférence à 1 % en poids dans l'eau, et comportant au moins une chaîne grasse comprenant au moins 6 atomes de carbone ou comportant au moins deux groupements siloxane.

Selon un mode de réalisation particulier de l'invention, la composition comprend de plus une ou plusieurs huiles, pouvant être notamment choisies parmi les huiles non siliconées minérale, végétale, animale ou synthétique et les huiles de silicones.

Comme huiles non siliconées utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique / caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité ;

- les hydrocarbures linéaires ou ramifiés contenant au moins 6 atomes de carbone, d'origine minérale ou synthétique, tels que les alcanes inférieurs C₆-C₁₆ comme l'hexane, le dodécane, l'isododécane et l'isohexadécane, les hydrocarbures à plus de 16 atomes de carbone comme les huiles de paraffine, et leurs dérivés, la vaseline, l'huile de vaseline, les polydécènes, le polyisobutène hydrogéné tel que Parléam^{®} ;
- les huiles fluorées partiellement hydrocarbonées ; comme huiles fluorées, on peut citer aussi le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M.
- les esters liquides d'acides et / ou d'alcool gras différents des triglycérides, et en particulier ceux pour lesquels l'acide et / ou l'alcool sont insaturés ou ramifiés, on peut utiliser en particulier le myristate d'isopropyle.

Parmi les huiles de silicones utilisables, on peut citer les polydialkylsiloxanes volatiles linéaires ayant 2 à 9 atomes de silicium et présentant une viscosité inférieure ou égale à 5.10⁻⁶ m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25 °C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶ m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SIO_{1/2}, RSiO_{3/2} et SiO_{4/2}
dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl / triméthyl siloxane.

Selon un mode de réalisation particulier de l'invention, la ou les huiles sont choisies parmi les alcanes linéaires ou ramifiés en C₆-C₁₆, les hydrocarbures de plus de 16 atomes de carbone, les triglycérides liquides d'acides gras comportant de 6 à 30 atomes de carbone, les huiles fluorées partiellement hydrocarbonées, les esters liquides d'acides et / ou d'alcool gras différents des triglycérides et les huiles de silicone.

Par milieu cosmétiquement acceptable, on entend, au sens de la présente invention, un milieu compatible avec les fibres kératiniques, et en particulier les fibres kératiniques humaines telles que les cheveux.

Le milieu cosmétiquement acceptable de la composition conforme à la présente invention comprend généralement de l'eau et / ou un ou plusieurs solvants organiques hydrosolubles. A titre de solvants organiques hydrosolubles, on peut par exemple citer les alcanols inférieurs en C₁-C₄, tels que l'éthanol et l'isopropanol ; les alcools aromatiques comme l'alcool benzylique ou le phénoxyéthanol ; les polyols ou éthers de polyols tels que les éthers monométhylique, monoéthylique et monobutylique d'éthylèneglycol, le propylèneglycol ou ses éthers tels que le monométhyléther de propylèneglycol, le butylèneglycol, le dipropylèneglycol ainsi que les alkyléthers de diéthylèneglycol comme le monoéthyléther ou le monobutyléther du diéthylèneglycol, ou encore le glycérol ; ainsi que leurs mélanges.

Les solvants hydrosolubles sont, de préférence, présents dans des proportions comprises entre 0,1 et 35 % en poids environ par rapport au poids total de la composition oxydante, et encore plus préférentiellement entre 1 et 40 % en poids environ.

La composition conforme à l'invention peut également comprendre des composés additionnels utilisés classiquement en cosmétique. Ces composés peuvent notamment être choisis parmi les polymères épaississants ou stabilisants, les polymères conditionneurs non siliconés, les chélatants, ainsi que les parfums.

Les compositions de l'invention contiennent un ou plusieurs amides d'acides gras en C₈-C₃₀ éventuellement oxyalkylénés. Ces amides peuvent être présents dans les compositions de l'invention à une teneur allant de 0,1 à 10 %, encore plus préférentiellement de 0,5 à 8 %, mieux de 1 à 5 % du poids total de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition selon l'invention peut se présenter sous des formes diverses, telles que sous forme de crème, de gel, de lait, de lotion, de mousse, ou sous toute autre forme appropriée pour réaliser le traitement des fibres kératiniques, et notamment des fibres kératiniques humaines telles que les cheveux. De préférence, elle se présente sous la forme d'une crème ou d'un lait.

Le pH de la composition oxydante selon l'invention varie de 1,5 à 4,5, et de préférence de 2 à 3,5. Il peut être ajusté par ajout d'agents acidifiants tels que l'acide chlorhydrique, l'acide acétique, l'acide lactique, l'acide borique, l'acide citrique et l'acide phosphorique ou d'agents acidifiants en présence d'agents alcalins.

Un autre objet de l'invention est un procédé de coloration ou de décoloration des fibres kératiniques comprenant l'application sur les fibres kératiniques d'une composition oxydante telle que définie précédemment.

La composition oxydante conforme à l'invention peut par exemple être utilisée dans un procédé de teinture des fibres kératiniques, et en particulier des fibres kératiniques humaines telles que les cheveux.

Le procédé de teinture des fibres kératiniques conforme à l'invention met en oeuvre une composition colorante comprenant dans un support approprié pour la teinture des fibres Kératiniques un ou plusieurs colorants directs et / ou un ou plusieurs colorants d'oxydation et une composition oxydante telle que définie ci-dessus.

Selon ce procédé, on applique sur les fibres kératiniques la composition colorante, la couleur étant révélée à pH acide, neutre ou alcalin à l'aide d'une composition oxydante selon l'invention qui est appliquée simultanément ou séquentiellement, avec ou sans rinçage intermédiaire.

Selon une forme de mise en oeuvre particulièrement préférée du procédé de teinture selon l'invention, on mélange, au moment de l'emploi, la composition colorante avec une composition oxydante selon l'invention. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

Le ou les colorants directs peuvent être choisis parmi les colorants directs classiquement utilisés en coloration directe. A titre d'exemples, ces colorants directs sont choisis parmi les colorants nitrés de la série benzénique, les colorants directs azoïques, les colorants directs méthiniques, les colorants directs quinoniques, les colorants directs aziniques, les colorants directs triarylméthaniques, les colorants directs indoaminiques, les colorants directs naturels. Ces colorants directs peuvent être de nature non ionique, anionique ou cationique.

Parmi les colorants directs benzéniques, on peut citer le 1,4-diamino-2-nitrobenzène, le 1-amino-2-nitro-4-(β-hydroxyéthylamino)-benzène, le 1-amino-2-nitro-4-bis(β-hydroxyéthyl)-aminobenzène, le 1,4-bis(β-hydroxyéthylamino)-2-nitro-benzène, le 1-[β-hydroxyéthylamino-2-nitro-4-bis-(β-hydroxyéthylamino)-benzène, le 1-β-hydroxyéthylamino-2-nitro-4-amino-benzène, le 1-β-hydroxyéthylamino-2-nitro-4-(éthyl)([β-hydroxyéthyl)-aminobenzène, le 1-amino-3-méthyl-4-β-hydroxyéthylamino-6-nitro-benzène, le 1-amino-2-nitro-4-β-hydroxyéthylamino-5-chloro-benzène, le 1,2-diamino-4-nitro-benzène, le 1-amino-2-β-hydroxyéthylamino-5-nitro-benzène, le 1,2-bis-(β-hydroxyéthylamino)-4-nitro-benzène, le 1-amino-2-[tris-(hydroxyméthyl)-méthylamino]-5-nitro-benzène, le 1-hydroxy-2-amino-5-nitro-benzène, le 1-hydroxy-2-amino-4-nitro-benzène, le 1-hydroxy-3-nitro-4-amino-benzène, le 1-hydroxy-2-amino-4,6-dinitro-benzène, le 1-β-hydroxyéthyloxy-2-β-hydroxyéthylamino-5-nitro-benzène, le 1-méthoxy-2-β-hydroxyéthylamino-5-nitro-benzène, le 1-β-hydroxyéthyloxy-3-méthylamino-4-nitrobenzène, le 1-β,γ-dihydroxypropyloxy-3-méthylamino-4-nitro-benzène, le 1-β-hydroxyéthylamino-4-β,γ-dihydroxypropyloxy-2-nitro-benzène, le 1-β,γ-dihydroxypropylamino-4-trifluorométhyl-2-nitro-benzène, le 1-β-hydroxyéthylamino-4-trifluorométhyl-2-nitro-benzène, le 1-β-hydroxyéthylamino-3-méthyl-2-nitro-benzène, le 1-β-aminoéthylamino-5-méthoxy-2-nitro-benzène, le 1-hydroxy-2-chloro-6-éthylamino-4-nitro-benzène, le 1-hydroxy-2-chloro-6-amino-4-nitro-benzène, le 1-hydroxy-6-[bis-(β-hydroxyéthyl)-amino]-3-nitro-benzène, le 1-β-hydroxyéthylamino-2-nitro-benzène, le 1-hydroxy-4-β-hydroxyéthylamino-3-nitro-benzène.

Parmi les colorants directs azoïques, on peut citer les colorants azoïques cationiques décrits dans les demandes de brevets WO 95/15144, WO-95/01772, EP 0 714 954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369 et FR 2 844 269 dont le contenu fait partie intégrante de l'invention.

Parmi ces composés, on peut tout particulièrement citer le chlorure de 1,3-diméthyl-2-[[4-(diméthylamino)phényl]azo]-1 H-Imidazolium, le chlorure de 1,3-diméthyl-2-[(4-aminophényl)azo]-1H-Imidazolium, le méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants : la 1-N-méthylmorpholiniumpropylamino-4-hydroxyanthraquinone, la 1-aminopropylamino-4-méthylaminoanthraquinone, la 1-aminopropylaminoanthraquinone, la 5-β-hydroxyéthyl-1,4-diaminoanthraquinone, la 2-aminoéthylaminoanthraquinone, la 1,4-bis-(β,γ-dihydroxypropylamino)-anthraquinone.

Parmi les colorants aziniques, on peut citer les composés suivants : Basic Blue 17, Basic Red 2.

Parmi les colorants triarylméthaniques, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques, on peut citer les composés suivants : la 2-β-hydroxyéthlyamino-5-[bis-(β-4-hydroxyéthyl)amino]anilino-1,4-benzoquinone, la 2-β-hydroxyéthylamino-5-(2'-méthoxy-4'-amino)anilino-1,4-benzoquinone, la 3-N(2'-Chioro-4'-hydroxy)phényl-acétylamino-6-méthoxy-1,4-benzoquinone imine, la 3-N(3'-Chloro-4'-méthylamino)phényl-uréido-6-méthyl-1,4-benzoquinone imine, la 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Le ou les colorants directs sont généralement présents dans la composition colorante en quantité comprise entre 0,001 et 20 % en poids environ du poids total de la composition, et encore plus préférentiellement entre 0,005 et 10 % en poids environ.

Le ou les colorants d'oxydation peuvent être choisis parmi les bases d'oxydation et les coupleurs conventionnellement utilisés dans le domaine de la coloration.

A titre d'exemples de bases d'oxydation, on peut citer les para-phénylènediamines, les bases doubles, les para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bases doubles, on peut citer, à titre d'exemples, les bis-phénylalkylènediamines et les bis-para-aminophénols.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

A titre de dérivés pyrazoliques, on peut aussi citer les diamino-N,N-dihydropyrazopyrazolones et notamment celles décrites dans la demande FR 2 886 136 telles que les composés suivants et leurs sels d'addition.

Parmi ces composés, les préférés sont les suivants :
2,3-Diamino-6,7-dihydro-1H,5H-pyrazolo[1,2-a]pyrazol-1-one,
2-Amino-3-éthylamino-6,7-dihydro-1H,5H-pyrazolo[1,2-a]pyrazol-1-one,
2-Amino-3-isopropylamino-6,7-dihydro-1H,5H-pyrazolo[1,2-a]pyrazol-1-one,
2-Amino-3-(pyrrolidin-1-yl)6,7-dihydro-1H,5H-pyrazolo[1,2-a]pyrazol-1-one,
4,5-diamino-1,2-diméthyl-1,2-dihydro-pyrazol-3-one,
4,5-diamino-1,2-diéthyl-1,2-dihydro-pyrazol-3-one,
4,5-diamino-1,2-di-(2-hydroxyéthyl)-1,2-dihydro-pyrazol-3-one,
2-amino-3-(2-hydroxyéthyl)amino-6,7-dihydro-1H,5H-pyrazolo[1,2-a]pyrazol-1-one,
2-amino-3-diméthylamino-6,7-dihydro-1H,5H-pyrazolo[1,2-a]pyrazol-1-one,
2,3-diamino-5,6,7,8-tétrahydro-1H,6H-pyridazino[1,2-a]pyrazol-1-one,
4-Amino-1,2-diethyl-5-(pyrrolidin-1-yl)-1,2-dihydro-pyrazol-3-one,
4-Amino-5-(3-dimethylamino-pyrrolidin-1-yl)-1,2-diethyl-1,2-dihydro-pyrazol-3-one,
2,3-diamino-6-hydroxy-6,7-dihydro-1H,5H-pyrazolo[1,2-a]pyrazol-1-one.

A titre de bases hétérocycliques, on utilisera préférentiellement le 4,5-diamino 1-(β-hydroxyéthyl) pyrazole, la 2,3-diamino-6,7-dihydro-1H,5H-pyrazolo[1,2-a]pyrazol-1-one et leurs sels d'addition.

La ou les bases d'oxydation sont généralement présentes dans la composition colorante en quantité comprise entre 0,001 et 10 % en poids environ du poids total de la composition, de préférence entre 0,005 et 6 % en poids environ.

A titre d'exemples de coupleurs, on peut citer les méta-phénylènediamines, les méta-aminophénols, les méta-diphénols, les coupleurs naphtaléniques, les coupleurs hétérocycliques et leurs sels d'addition.

On peut notamment citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Le ou les coupleurs sont généralement présents dans la composition colorante en quantité comprise entre 0,001 et 10 % en poids environ du poids total de la composition, de préférence entre 0,005 et 6 % en poids environ.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telle que la soude, la potasse, l'ammoniaque, les amines ou les alcanolamines.

La composition oxydante selon l'invention peut également être utilisée dans un procédé de décoloration des fibres kératiniques, et en particulier des fibres kératiniques humaines telles que les cheveux.

Le procédé de décoloration selon l'invention comprend une étape d'application sur les fibres kératiniques d'une composition décolorante comprenant de préférence de l'eau oxygénée en milieu alcalin après mélange extemporané. Classiquement, une deuxième étape du procédé de décoloration selon l'invention est une étape de rinçage des fibres kératiniques.

La composition décolorante appliquée sur les fibres kératiniques peut être obtenue par mélange d'une composition oxydante selon l'invention avec une composition aqueuse ou anhydre contenant de préférence un ou plusieurs agents alcalins. Ces agents alcalins peuvent être choisis parmi l'ammoniaque, les alcanolamines dont le monoéthanolamine, les acides aminés et en particulier les acides aminés basiques comme la lysine ou l'arginine, les carbonates ou bicarbonates alcalins, les hydroxydes alcalins, le carbonate de guanidine et leurs mélanges. La composition anhydre peut être pulvérulente ou sous forme de pate et dans les deux cas contient de préférence un ou plusieurs sels peroxygénés, et en particulier un ou plusieurs persulfates. La composition anhydre sous forme de pate contient de plus un ou plusieurs liquides inertes organiques.

La présente invention a également pour objet l'utilisation pour la coloration ou la décoloration des fibres kératiniques, et en particulier des fibres kératiniques humaines telles que les cheveux, d'une composition oxydante telle que définie ci-dessus.

La présente invention a notamment pour objet l'utilisation pour la teinture des fibres kératiniques, et en particulier des fibres kératiniques humaines telles que les cheveux, d'une composition oxydante telle que définie ci-dessus.

La présente invention a également pour objet l'utilisation pour la décoloration des fibres kératiniques, et en particulier des fibres kératiniques humaines telles que les cheveux, d'une composition oxydante telle que définie ci-dessus.

Les exemples suivants illustrent l'invention sans présenter un caractère limitatif.

### EXEMPLES

Les compositions suivantes sont préparées :

| Composition | A | B |
|---|---|---|
| Acide diéthylène triamine pentacétique, sel pentasodique en solution aqueuse à 40 % | 0,15 g | 0,15 g |
| Peroxyde d'hydrogène en solution à 50 % (eau oxygénée 200 volumes) | 12 g | 12 g |
| Stannate de sodium | 0,04 g | 0,04 g |
| Pyrophosphate tétra-sodique, 10 H₂O | 0,03 g | 0,03 g |
| Polycondensat tétraméthyl hexaméthylènediamine dichloro 1,3-propylène en solution aqueuse vendu sous la dénomination MEXOMERE PO par la société Chimex | 0,25 g | 0,25 g |
| Chlorure de poly di-méthyl di-allyl ammonium dans l'eau à 40 % non stabilisé vendu sous la dénomination Merquat 100 par la société Nalco | 0,50 g | 0,50 g |
| Eau désionisée | 49,13 g | 50,73 g |
| Huile de vaseline | 25 g | 25 g |
| Glycérine | 0,50 g | 0,50 g |
| Alcool stéarylique 30/70 | 8 g | 8 g |
| Ceteareth-33 | 3g | 1,40 g |
| Amide d'acides de colza oxyéthyléné (4 OE) protégé | 1,30 g | 1,30 g |
| Vitamine E | 0,10 g | 0,10 g |
| Acide phosphorique q.s. | pH 2 | pH 2 |

La composition B n'est pas stable, une séparation de phase intervient 15 jours après la formulation, à 45 °C. Dans cette composition, le rapport pondéral alcools gras / alcools gras oxyalkylénés est de 5,7.

La composition A est stable, y compris après 2 mois à 45 °C. Dans cette composition, le rapport pondéral alcools gras / alcools gras oxyalkylénés est de 2,7.

## Revendications

1. Composition oxydante pour la coloration ou la décoloration des fibres kératiniques comprenant, dans un milieu cosmétiquement acceptable :
- un ou plusieurs alcools gras ;
- un ou plusieurs alcools gras oxyalkylénés ;
- un ou plusieurs agents oxydants ;
- au moins 10 % en poids du poids total de la composition d'une ou plusieurs huiles différentes des alcools gras ; le rapport pondéral alcools gras / alcools gras oxyalkylénés étant inférieur ou égal à 5 le pH de cette composition étant compris entre 1,5 et 4,5 et
- un ou plusieurs amides d'acides gras en C8-C30 éventuellement oxyalkylénées.

2. Composition selon la revendication 1 dans laquelle le ou les alcools gras sont choisis parmi les monoalcools non (poly)oxyalkylénés et non (poly)glycérolés, comprenant de 14 à 22 atomes de carbone, saturés.

3. Composition selon la revendication 1 ou 2, dans laquelle la teneur en alcool(s) gras est comprise entre 3 et 25 % en poids par rapport au poids de la composition.

4. Composition selon l'une quelconque des revendications précédentes dans laquelle le ou les alcools gras oxyalkylénés sont linéaires ou ramifiés, saturés ou insaturés, et comportent 8 à 40 atomes de carbone et 1 à 250 groupements oxyde d'éthylène et / ou oxyde de propylène.

5. Composition selon la revendication 4 dans laquelle le ou les alcools gras oxyalkylénés sont linéaires ou ramifiés, saturés ou insaturés, et comportent 10 à 20 atomes de carbone et 2 à 40 groupements oxyde d'éthylène.

6. Composition selon l'une quelconque des revendications précédentes dans laquelle le ou les alcools gras oxyalkylénés représentent de 0,05 à 50 % en poids du poids total de la composition.

7. Composition selon l'une quelconque des revendications précédentes dans laquelle le ou les agents oxydants sont choisis parmi le peroxyde d'hydrogène, le peroxyde d'urée, les bromates alcalins, les polythionates, les persels.

8. Composition selon l'une quelconque des revendications précédentes dans laquelle le ou les agents oxydants représentent de 0,1 à 50 % en poids par rapport au poids total de la composition.

9. Composition selon l'une quelconque des revendications précédentes dans laquelle le rapport pondéral alcools gras / alcools gras oxyalkylénés va de 0,1 à 5.

10. Composition selon l'une quelconque des revendications précédentes comprenant au moins 15 % en poids du poids total de la composition d'une ou plusieurs huiles différentes des alcools gras.

11. Composition selon l'une quelconque des revendications précédentes dans laquelle la ou les huiles sont choisies parmi les alcanes linéaires ou ramifiés en C₆-C₁₆, les hydrocarbures de plus de 16 atomes de carbone, les triglycérides liquides d'acides gras comportant de 6 à 30 atomes de carbone, les huiles fluorées partiellement hydrocarbonées, les esters liquides d'acides et / ou d'alcool gras différents des triglycérides et les huiles de silicone.

12. Procédé de coloration ou de décoloration des fibres kératiniques comprenant l'application sur les fibres kératiniques d'une composition oxydante telle que définie à l'une quelconque des revendications 1 à 11.

13. Utilisation pour la coloration ou la décoloration des fibres kératiniques d'une composition oxydante telle que définie à l'une quelconque des revendications 1 à 11.

## Patentansprüche

1. Oxidierende Zusammensetzung zum Färben oder Bleichen von Keratinfasern, die in einem kosmetisch unbedenklichen Medium
- einen oder mehrere Fettalkohole;
- einen oder mehrere oxyalkylenierte Fettalkohole;
- ein oder mehrere Oxidationsmittel;
- mindestens 10 Ges.-%, bezogen auf das Gesamtgewicht der Zusammensetzung, eines oder mehrerer Öle, die von Fettalkoholen verschieden sind; wobei das Gewichtsverhältnis von Fettalkoholen zu oxyalkylenierten Fettalkoholen kleiner gleich 5 ist, wobei der pH-Wert dieser Zusammensetzung zwischen 1,5 und 4,5 liegt, und
- ein oder mehrere gegebenenfalls oxyalkylenierte C8-C30-Fettsäureamide
umfasst.

2. Zusammensetzung nach Anspruch 1 , wobei der bzw. die Fettalkohole aus nicht (poly)oxyalkylenierten und nicht (poly)glycerinierten gesättigten Monoalkoholen mit 14 bis 22 Kohlenstoffatomen ausgewählt sind.

3. Zusammensetzung nach Anspruch 1 oder 2, wobei der Gehalt an Fettalkohol(en) zwischen 3 und 25 Gew.-%, bezogen auf das Gewicht der Zusammensetzung, liegt.

4. Zusammensetzung nach einem der vorhergehenden Ansprüche, wobei der bzw. die oxyalkylenierten Fettalkohole linear oder verzweigt und gesättigt oder ungesättigt sind und 8 bis 40 Kohlenstoffatome und 1 bis 250 Ethylenoxid- und/oder Propylenoxidgruppen enthalten.

5. Zusammensetzung nach Anspruch 4, wobei der bzw. die oxyalkylenierten Fettalkohole linear oder verzweigt und gesättigt oder ungesättigt sind und 10 bis 20 Kohlenstoffatome und 2 bis 40 Ethylenoxidgruppen enthalten.

6. Zusammensetzung nach einem der vorhergehenden Ansprüche, wobei der bzw. die oxyalkylenierten Fettalkohole 0,05 bis 50 Gew.-%, bezogen auf das Gesamtgewicht der Zusammensetzung, ausmachen.

7. Zusammensetzung nach einem der vorhergehenden Ansprüche, wobei das bzw. die Oxidationsmittel aus Wasserstoffperoxid, Harnstoffperoxid, Alkalibromaten, Polythionaten und Persalzen ausgewählt sind.

8. Zusammensetzung nach einem der vorhergehenden Ansprüche, wobei das bzw. die Oxidationsmittel 0,1 bis 50 Gew.-%, bezogen auf das Gesamtgewicht der Zusammensetzung, ausmachen.

9. Zusammensetzung nach einem der vorhergehenden Ansprüche, wobei das Gewichtsverhältnis von Fettalkoholen zu oxyalkylenierten Fettalkoholen im Bereich von 0,1 bis 5 liegt.

10. Zusammensetzung nach einem der vorhergehenden Ansprüche, umfassend mindestens 15 Gew.-%, bezogen auf das Gesamtgewicht der Zusammensetzung, eines oder mehrerer Öle, die von Fettalkoholen verschieden sind.

11. Zusammensetzung nach einem der vorhergehenden Ansprüche, wobei das bzw. die Öle aus linearen oder verzweigten C₆-C₁₆-Alkanen, Kohlenwasserstoffen mit mehr als 16 Kohlenstoffatomen, flüssigen Triglyceriden von Fettsäuren mit 6 bis 30 Kohlenstoffatomen, teilfluorierten Kohlenwasserstoffölen und flüssigen Estern von Fettsäuren und/oder Fettalkoholen, die von Triglyceriden und Silikonölen verschieden sind, ausgewählt sind.

12. Verfahren zum Färben oder Bleichen von Keratinfasern, bei dem man auf die Keratinfasern eine oxidierende Zusammensetzung gemäß einem der Ansprüche 1 bis 11 aufbringt.

13. Verwendung einer oxidierenden Zusammensetzung gemäß einem der Ansprüche 1 bis 11 zum Färben oder Bleichen von Keratinfasern.

## Claims

1. Oxidizing composition for dyeing or bleaching keratin fibres comprising, in a cosmetically acceptable medium:
- one or more fatty alcohols;
- one or more oxyalkylenated fatty alcohols;
- one or more oxidizing agents;
- at least 10 wt%, relative to the total weight of the composition, of one or more oils other than fatty alcohols; the fatty alcohols/oxyalkylenated fatty alcohol weight ratio being less than or equal to 5, the pH of this composition being between 1.5 and 4.5; and
- one or more optionally oxyalkylenated C₈-C₃₀ fatty acid amides.

2. Composition according to Claim 1, in which the fatty alcohol(s) is (are) selected from non-(poly)oxyalkylenated and non-(poly)glycerolated saturated monoalcohols containing from 14 to 22 carbon atoms.

3. Composition according to Claim 1 or 2, in which the content of fatty alcohol(s) is between 3 and 25 wt% relative to the weight of the composition.

4. Composition according to any one of the preceding claims, in which the oxyalkylenated fatty alcohol(s) is (are) linear or branched, and saturated or unsaturated, and contain(s) 8 to 40 carbon atoms and 1 to 250 ethylene oxide and/or propylene oxide groups.

5. Composition according to Claim 4, in which the oxyalkylenated fatty alcohol(s) is (are) linear or branched, saturated or unsaturated, and contain(s) 10 to 20 carbon atoms and 2 to 40 ethylene oxide groups.

6. Composition according to any one of the preceding claims, in which the oxyalkylenated fatty alcohol(s) represent(s) from 0.05 to 50 wt% relative to the total weight of the composition.

7. Composition according to any one of the preceding claims, in which the oxidizing agent(s) is (are) selected from hydrogen peroxide, urea peroxide, alkaline bromates, polythionates and persalts.

8. Composition according to any one of the preceding claims, in which the oxidizing agent(s) represent (s) from 0.1 to 50 wt% relative to the total weight of the composition.

9. Composition according to any one of the preceding claims, in which the fatty alcohol/oxyalkylenated fatty alcohol weight ratio ranges from 0.1 to 5.

10. Composition according to any one of the preceding claims, comprising at least 15 wt% of one or more oils other than fatty alcohols, relative to the total weight of the composition.

11. Composition according to any one of the preceding claims, in which the oil(s) is (are) selected from C₆-C₁₆ linear or branched alkanes, hydrocarbons containing more than 16 carbon atoms, liquid triglycerides of fatty acids containing from 6 to 30 carbon atoms, fluorinated, partially hydrocarbon-based oils, liquid esters of fatty acids and/or of fatty alcohols other than the triglycerides and silicone oils.

12. Method of dyeing or bleaching keratin fibres, comprising the application of an oxidizing composition as defined in any one of Claims 1 to 11 on the keratin fibres.

13. Use of an oxidizing composition as defined in any one of Claims 1 to 11 for dyeing or bleaching keratin fibres.
